# EUROPEAN PATENT APPLICATION

(11) **EP 4 477 731 A1**
(43) Date of publication of application: **18.12.2024**
(21) Application number: 22966202.8
(22) Date of filing: 24.11.2022
(51) Int. Cl.: C10G 59/02, C10G 11/00, C07C 15/08, C07C 11/04

(54) **DEVICE AND METHOD FOR PREPARING AROMATIC HYDROCARBONS BY COUPLING NAPHTHA AND METHANOL**

(71) Applicant: China Shenhua Coal to Liquid and Chemical Co., Ltd, Beijing 100011 (CN); Dalian Institute of Chemical Physics, CAS, Dalian, Liaoning 116023 (CN)
(72) Inventor: YE, Mao, Dalian, Liaoning 116023 (CN); YAN, Guochun, Beijing 100011 (CN); LIU, Zhongmin, Dalian, Liaoning 116023 (CN); ZHANG, Jiming, Beijing 100011 (CN); ZHANG, Tao, Dalian, Liaoning 116023 (CN); WEN, Liang, Beijing 100011 (CN); ZHANG, Jinling, Dalian, Liaoning 116023 (CN); ZHANG, Yanbin, Beijing 100011 (CN); JIA, Jinming, Dalian, Liaoning 116023 (CN); XU, Haibo, Beijing 100011 (CN); TANG, Hailong, Dalian, Liaoning 116023 (CN); YIN, Tian, Beijing 100011 (CN); ZHANG, Cheng, Dalian, Liaoning 116023 (CN); JIAO, Yanzhong, Beijing 100011 (CN); WANG, Xiangao, Dalian, Liaoning 116023 (CN); XIANG, Lipeng, Beijing 100011 (CN); MA, Xiangang, Dalian, Liaoning 116023 (CN); LIN, Wei, Beijing 100011 (CN); WANG, Jing, Dalian, Liaoning 116023 (CN); ZHAO, Yan, Beijing 100011 (CN); YAO, Shunyu, Beijing 100011 (CN); ZHAO, Zonglai, Beijing 100011 (CN)
(74) Representative: Bergenstråhle & Partners AB
(86) International application number: PCT/CN2022/134183
(87) International publication number: WO 2024/108511

(57) **Abstract**

The present application discloses a device and method for preparing aromatic hydrocarbons by coupling naphtha and methanol. By adopting the device, under an action of a catalyst, the naphtha and the methanol react to generate product gas with aromatic hydrocarbons and a low-carbon olefin as main components. By the method of the application, linear-chain and branched-chain aliphatic hydrocarbons can be efficiently converted into aromatic hydrocarbons in a highly selective mode, a yield of p-xylene is also increased through a methylation reaction of aromatic hydrocarbons, and a content of p-xylene in a xylene mixture is greater than 75wt%. By a naphtha and methanol coupled aromatic hydrocarbon preparation reactor in the application, the yield of the p-xylene is increased by controlling a process of a cascade reaction (naphtha→benzene and toluene→p-xylene), and in addition, heat is provided in situ for a naphtha and methanol coupled aromatic hydrocarbon preparation reaction through a methylation reaction of benzene and toluene with methanol, so that self-heating balance is achieved.

## Description

### TECHNICAL FIELD

The present application relates to a fluidized bed device and a method for using the device, belongs to the technical field of chemical industry, and particularly relates to a device and method for preparing aromatic hydrocarbons by coupling naphtha and methanol.

### BACKGROUND

Aromatic hydrocarbons (benzene, toluene and xylene, collectively referred to as BTX) are important organic chemical raw materials, wherein p-xylene (PX) is the most concerned product of aromatic hydrocarbons, which is mainly used for producing polyesters such as terephthalic acid (PTA), polyethylene terephthalate (PET), polybutylene terephthalate (PBT) and polytrimethylene terephthalate (PTT). In recent years, a yield and a consumption of p-xylene in China have been increasing continuously. In 2021, a total import volume of PX in China was about 13.65 million tons, and a foreign-trade dependence degree was about 38%.

Naphtha catalytic reforming technology is a main technical route for producing aromatic hydrocarbons. The composition of naphtha is very complex, naphtha is not only a main raw material for catalytic reforming, but also a main raw material for ethylene preparation by cracking, and the composition of naphtha plays an important role in economic benefits of a device. Generally speaking, a high potential content and a moderate distillation range of the raw material aromatic hydrocarbons are beneficial for catalytic reforming. However, high contents of linear-chain and branched-chain aliphatic hydrocarbons and low contents of naphthenic hydrocarbon and aromatic hydrocarbon are suitable for ethylene preparation by cracking. Usually, in order to make full use of naphtha resources and improve economic benefits, it is necessary to separate the linear-chain and branched-chain aliphatic hydrocarbons in the naphtha from the naphthenic hydrocarbon and the aromatic hydrocarbons first, the former are used as raw materials for ethylene production, and the latter are used as raw materials for a catalytic reforming device.

The distillation range of the fractoins of naphtha is wide, it is difficult to efficiently separate the linear-chain and branched-chain aliphatic hydrocarbons from the naphthenic hydrocarbon and the aromatic hydrocarbons by a general separation method, and in addition, it is difficult to convert the linear-chain and branched-chain aliphatic hydrocarbons into the aromatic hydrocarbons by the catalytic reforming technology. The naphtha raw material used for catalytic reforming generally needs to be distilled to separate topped oil with a boiling point below 60°C, so as to increase a potential content of the raw material aromatic hydrocarbons for catalytic reforming. However, a fraction with a boiling point above 60°C still contains a large number of linear-chain and branched-chain aliphatic hydrocarbons difficult to be converted into aromatic hydrocarbons. Therefore, converting the linear-chain and branched-chain aliphatic hydrocarbons into aromatic hydrocarbons in a highly selective mode has always been a hot and difficult point in the development of a technology for preparing aromatic hydrocarbons from the naphtha.

Due to the limitation of thermodynamic equilibrium, p-xylene in a xylene mixture produced by the naphtha catalytic reforming device only accounts for ~24%, and it is necessary to further increase a yield of the p-xylene through an isomerization-separation technology. Therefore, increasing the content of the p-xylene in the xylene mixture is an important way to reduce energy consumption for the production of p-xylene.

### SUMMARY

A naphtha molecule contains only a small amount of methyl (methyl/benzene ring = 1. 3 (molar ratio)), and a molecular structure of the naphtha molecule determines that a large amount of benzene is inevitably by-produced in a catalytic reforming/aromatics complex device.

Methanol aromatization is a new aromatic hydrocarbon preparation process, but compared with aromatic hydrocarbons, there are excess hydrogen atoms in a methanol molecule, so that a large amount of alkane and a large amount of hydrogen are inevitably by-produced in aromatic hydrocarbon preparation with methanol. According to a molecular structure and a reaction mechanism, the methanol may provide methyl for aromatic hydrocarbons, so as to increase yields of toluene and xylene, which provides a new technical route for preparing aromatic hydrocarbons by coupling naphtha and methanol.

In one aspect of the present application, a device capable of preparing aromatic hydrocarbons with naphtha and methanol as raw materials is provided, and the device increases a content of p-xylene in mixed xylene and reduces energy consumption for separation.

The naphtha in the present application comprises components of C₄-C₁₂ linear-chain and branched-chain aliphatic hydrocarbons, naphthenic hydrocarbons and aromatic hydrocarbons.

The aromatic hydrocarbons in the present application refer to benzene, toluene and xylene, collectively called BTX.

The device for preparing aromatic hydrocarbons by coupling naphtha and methanol comprises a light hydrocarbon aromatization reactor and a naphtha and methanol coupled aromatic hydrocarbon preparation reactor; wherein,
the light hydrocarbon aromatization reactor is used for introducing raw materials and a high-temperature catalyst; and at least one outlet of the light hydrocarbon aromatization reactor is connected to the naphtha and methanol coupled aromatic hydrocarbon preparation reactor for conveying the catalyst and generated light hydrocarbon aromatization product gas to the naphtha and methanol coupled aromatic hydrocarbon preparation reactor; and
the naphtha and methanol coupled aromatic hydrocarbon preparation reactor is used for introducing naphtha and methanol and allowing the naphtha to make contact with the catalyst from the light hydrocarbon aromatization reactor to generate a BTX-containing product gas stream after reaction; and subjecting the methanol to a methylation reaction with benzene and toluene in the product gas stream to generate p-xylene.

When the catalyst enters the naphtha and methanol coupled aromatic hydrocarbon preparation reactor, a temperature of the catalyst is reduced to some extent, and at this time, the catalyst makes contact with the naphtha, which can eliminate a local high-temperature zone in the naphtha and methanol coupled aromatic hydrocarbon preparation reactor, thus effectively reducing a yield of low-carbon alkane and increasing a yield of aromatic hydrocarbons.

Preferably, the naphtha and methanol coupled aromatic hydrocarbon preparation reactor is at least divided into a first gas-solid separation zone and a naphtha and methanol coupled aromatic hydrocarbon preparation reaction zone from top to bottom, the two zones are communicated; and a naphtha and methanol coupled aromatic hydrocarbon preparation reactor distributor is provided in the naphtha and methanol coupled aromatic hydrocarbon preparation reaction zone, which comprises n sub-distributors, serial numbers of the sub-distributors are 1 to n sequentially from bottom to top, n≥2, wherein, a 1^{st} sub-distributor is used for introducing a naphtha raw material, and a 2^{nd} sub-distributor to an n^{th} sub-distributor are used for introducing a methanol raw material.

Preferably, n≤10.

Preferably, a gas-solid separation device I and a gas collection chamber I are provided in the first gas-solid separation zone; a gas outlet of the gas-solid separation device I is communicated with the gas collection chamber I; and an outlet of the gas collection chamber I is communicated with a product gas conveying pipe I, and the product gas conveying pipe I is used for outputting the BTX-containing product gas stream after gas-solid separation to a downstream working section.

Preferably, the gas collection chamber I is located on an inner top portion of a naphtha and methanol coupled aromatic hydrocarbon preparation reactor shell.

Preferably, the gas-solid separation device I is one or more groups of gas-solid cyclone separators, and each group of gas-solid cyclone separators comprises a first-stage gas-solid cyclone separator and a second-stage gas-solid cyclone separator.

Preferably, the light hydrocarbon aromatization reactor is at least divided into a second gas-solid separation zone and a light hydrocarbon aromatization reaction zone from top to bottom to form a bed reactor, and the two zones are communicated; and the second gas-solid separation zone is provided with a gas-solid separation device II and a gas collection chamber II, a gas outlet of the gas-solid separation device II is communicated with the gas collection chamber II, and a bed reactor distributor is provided on an inner lower portion of the light hydrocarbon aromatization reaction zone for introducing a bed reactor raw material.

Preferably, the gas collection chamber II is provided on an inner top portion of the bed reactor.

Further, the bed reactor raw material comprises C₄ and C₅ hydrocarbons, and the C₄ and C₅ hydrocarbons refer to hydrocarbons with 4 and 5 C atoms.

Preferably, the bed reactor raw material comprises C₃, C₄ and C₅ hydrocarbons, and the C₃, C₄ and C₅ hydrocarbons refer to hydrocarbons with 3, 4 and 5 C atoms.

Preferably, besides the bed reactor, the light hydrocarbon aromatization reactor further comprises a riser reactor, an outlet end of the riser reactor extends into an inner lower portion of the light hydrocarbon aromatization reaction zone, and a catalyst outlet of the gas-solid separation device II is arranged above the riser reactor.

Preferably, an inlet end of the riser reactor is also used for introducing the catalyst and a riser reactor raw material.

Preferably, the second gas-solid separation zone is communicated with the first gas-solid separation zone, and the light hydrocarbon aromatization reaction zone is communicated with the naphtha and methanol coupled aromatic hydrocarbon preparation reaction zone.

Preferably, the gas collection chamber II is communicated with the first gas-solid separation zone through a product gas conveying pipe II.

Preferably, a light hydrocarbon aromatization slide valve is provided on a pipeline connecting the naphtha and methanol coupled aromatic hydrocarbon preparation reaction zone and the light hydrocarbon aromatization reaction zone.

Preferably, a position of an outlet of the light hydrocarbon aromatization reaction zone is higher than a position of an inlet of the naphtha and methanol coupled aromatic hydrocarbon preparation reaction zone.

Preferably, a position of a catalyst inlet of the naphtha and methanol coupled aromatic hydrocarbon preparation reaction zone is located between the 1^{st} sub-distributor and the 2^{nd} sub-distributor.

Preferably, the gas-solid separation device II is a gas-solid cyclone separator.

Preferably, the device further comprises a regenerator, and at least one inlet of the light hydrocarbon aromatization reactor is connected to the regenerator for acquiring a high-temperature regenerated catalyst generated by the regenerator.

Preferably, an inlet end of a riser reactor of the light hydrocarbon aromatization reactor is communicated with the regenerator.

Preferably, the regenerator is at least divided into a third gas-solid separation zone and a regeneration zone from top to bottom, and the two zones are communicated; the third gas-solid separation zone is provided with a regenerator gas-solid separation device and a regenerator gas collection chamber; a gas outlet of the regenerator gas-solid separation device is communicated with the regenerator gas collection chamber; a flue gas conveying pipe is provided on the regenerator gas collection chamber; and a regenerator distributor is provided on an inner lower portion of the regeneration zone for introducing regeneration gas.

Preferably, the regeneration zone sequentially passes through a regenerator stripper and a regenerated slide valve to be connected to the riser reactor; and an inlet pipe of the regenerator stripper extends into a regenerator shell and is located above the regenerator distributor, and a catalyst outlet end of the regenerator gas-solid separation device is located above an opening end of the inlet pipe of the regenerator stripper.

Preferably, the regenerator gas collection chamber is located on an inner top portion of the regenerator shell.

Preferably, the regenerator gas-solid separation device is one or more groups of gas-solid cyclone separators, and each group of gas-solid cyclone separators comprises a first-stage gas-solid cyclone separator and a second-stage gas-solid cyclone separator.

Preferably, at least one outlet of the naphtha and methanol coupled aromatic hydrocarbon preparation reactor is also connected with an inlet of a regenerator for introducing a spent catalyst generated by a reaction of the naphtha and methanol coupled aromatic hydrocarbon preparation reactor into the regenerator, and the regenerator is used for introducing regeneration gas to convert the spent catalyst into a regenerated catalyst.

Preferably, the naphtha and methanol coupled aromatic hydrocarbon preparation reaction zone sequentially passes through a reactor stripper, a spent slide valve and a spent agent conveying pipe to be connected to an inlet of a regenerator; an inlet pipe of the reactor stripper extends into a naphtha and methanol coupled aromatic hydrocarbon preparation reactor shell and is located above the 1^{st} distributor, and a catalyst outlet end of the reactor gas-solid separation device is located above an opening end of an inlet pipe of the reactor stripper.

Preferably, an inlet of the regenerator is located in a regeneration zone and is provided on a regenerator shell.

In another aspect of the present application, a method for preparing aromatic hydrocarbons by coupling naphtha and methanol is provided, wherein the method comprises: preparing aromatic hydrocarbons by using the device for preparing aromatic hydrocarbons by coupling naphtha and methanol above and a catalyst.

Preferably, the catalyst is a metal molecular sieve bifunctional catalyst;
preferably, the metal molecular sieve bifunctional catalyst is a metal modified HZSM-5 zeolite molecular sieve;
metal for the metal modification is selected from at least one of La, Zn, Ga, Fe, Mo and Cr; and
a method for the metal modification comprises: placing the HZSM-5 zeolite molecular sieve in a metal salt solution, soaking, drying and roasting to obtain the metal modified HZSM-5 zeolite molecular sieve.

Further, the method comprises the following steps of:
introducing the raw materials and the high-temperature catalyst into the light hydrocarbon aromatization reactor to generate the light hydrocarbon aromatization product gas;
introducing the naphtha and the catalyst from the light hydrocarbon aromatization reactor into the naphtha and methanol coupled aromatic hydrocarbon preparation reactor to generate a BTX-containing product gas stream; and
introducing the methanol into the naphtha and methanol coupled aromatic hydrocarbon preparation reactor to be subjected to the methylation reaction with benzene and toluene in the BTX-containing product gas stream to generate p-xylene.

Preferably, a spent catalyst contained in all gas streams generated in the naphtha and methanol coupled aromatic hydrocarbon preparation reactor is removed through the gas-solid separation device I, and the gas streams enter the gas collection chamber I and then enter the downstream working section through the product gas conveying pipe I.

Preferably, the light hydrocarbon aromatization product gas comprises components of BTX, low-carbon olefins and Hz.

Preferably, besides the BTX, the BTX-containing product gas stream further comprises low-carbon olefins, hydrogen, low-carbon alkanes, combustible gases, heavy aromatic hydrocarbons and unconverted naphtha.

Preferably, the low-carbon olefins refer to ethylene and propylene;
the low-carbon alkanes refer to ethane and propane;
the combustible gases comprise methane and CO; and
the heavy aromatic hydrocarbons refer to an aromatic hydrocarbons with a number of carbon atoms in a molecule greater than or equal to 9.

Preferably, the naphtha is selected from at least one of direct coal liquefaction naphtha, indirect coal liquefaction naphtha, straight-run naphtha and hydrocracking naphtha.

Preferably, the naphtha also contains unconverted naphtha separated from the product gas stream, and the unconverted naphtha comprises main components of C₄-C₁₂ linear-chain and branched-chain aliphatic hydrocarbons and a naphthenic hydrocarbon.

Preferably, a carbon content in the spent catalyst is 1.0wt% to 3.0wt%.

Preferably, process conditions of the naphtha and methanol coupled aromatic hydrocarbon preparation reaction zone are: an apparent linear velocity of gas of 0.5 m/s to 2.0 m/s, a reaction temperature of 500°C to 600°C, a reaction pressure of 100 kPa to 500 kPa, and a bed density of 150 kg/m³ to 700 kg/m³.

Optionally, the apparent linear velocity of gas of the naphtha and methanol coupled aromatic hydrocarbon preparation reaction zone is independently selected from any value or a value in a range between any two of 0.5 m/s, 0.6 m/s, 0.7 m/s, 0.8 m/s, 0.9 m/s, 1.0 m/s, 1.1 m/s, 1.2 m/s, 1.3 m/s, 1.4 m/s, 1.5 m/s, 1.6 m/s, 1.7 m/s, 1.8 m/s, 1.9 m/s and 2.0 m/s.

Optionally, the reaction temperature of the naphtha and methanol coupled aromatic hydrocarbon preparation reaction zone is independently selected from any value or a value in a range between any two of 500°C, 520°C, 530°C, 540°C, 550°C, 560°C, 570°C, 580°C, 590°C and 600°C.

Optionally, the reaction pressure of the naphtha and methanol coupled aromatic hydrocarbon preparation reaction zone is independently selected from any value or a value in a range between any two of 100 kPa, 125 kPa, 150 kPa, 175 kPa, 200 kPa, 225 kPa, 250 kPa, 275 kPa, 300 kPa, 325 kPa, 350 kPa, 375 kPa, 400 kPa, 425 kPa, 450 kPa, 475 kPa and 500 kPa.

Optionally, the bed density of the naphtha and methanol coupled aromatic hydrocarbon preparation reaction zone is independently selected from any value or a value in a range between any two of 150 kg/m³, 200 kg/m³, 250 kg/m³, 300 kg/m³, 350 kg/m³, 400 kg/m³, 450 kg/m³, 500 kg/m³, 550 kg/m³, 600 kg/m³, 650 kg/m³ and 700 kg/m³.

Preferably, the light hydrocarbon aromatization product gas enters a gas-solid separation device II to remove a catalyst contained in the light hydrocarbon aromatization product gas, then enters a gas collection chamber II, and enters the first gas-solid separation zone of the naphtha and methanol coupled aromatic hydrocarbon preparation reactor through a product gas conveying pipe II; and
the catalyst in the light hydrocarbon aromatization reaction zone enters the naphtha and methanol coupled aromatic hydrocarbon preparation reactor through a light hydrocarbon aromatization slide valve.

Optionally, process conditions of the light hydrocarbon aromatization reaction zone are: an apparent linear velocity of gas of 0.5 m/s to 2.0 m/s, a reaction temperature of 550°C to 665°C, a reaction pressure of 100 kPa to 500 kPa, and a bed density of 150 kg/m³ to 700 kg/m³.

Optionally, the apparent linear velocity of gas of the light hydrocarbon aromatization reaction zone is independently selected from any value or a value in a range between any two of 0.5 m/s, 0.6 m/s, 0.7 m/s, 0.8 m/s, 0.9 m/s, 1.0 m/s, 1.1 m/s, 1.2 m/s, 1.3 m/s, 1.4 m/s, 1.5 m/s, 1.6 m/s, 1.7 m/s, 1.8 m/s, 1.9 m/s and 2.0 m/s.

Optionally, the reaction temperature of the light hydrocarbon aromatization reaction zone is independently selected from any value or a value in a range between any two of 550°C, 560°C, 570°C, 580°C, 590°C, 600°C, 610°C, 620°C, 630°C, 640°C, 650°C, 660°C and 665°C.

Optionally, the reaction pressure is independently selected from any value or a value in a range between any two of 100 kPa, 125 kPa, 150 kPa, 175 kPa, 200 kPa, 225 kPa, 250 kPa, 275 kPa, 300 kPa, 325 kPa, 350 kPa, 375 kPa, 400 kPa, 425 kPa, 450 kPa, 475 kPa and 500 kPa.

Optionally, the bed density is independently selected from any value or a value in a range between any two of 150 kg/m3, 200 kg/m³, 250 kg/m³, 300 kg/m³, 350 kg/m³, 400 kg/m³, 450 kg/m³, 500 kg/m³, 550 kg/m³, 600 kg/m³, 650 kg/m³ and 700 kg/m³.

Preferably, the method further comprises: introducing regeneration gas and a spent catalyst into a regenerator to obtain a high-temperature regenerated catalyst, and conveying the high-temperature regenerated catalyst to the light hydrocarbon aromatization reactor.

Preferably, the regeneration gas is introduced into a regeneration zone of the regenerator through a regenerator distributor.

Preferably, the regeneration gas is selected from at least one of oxygen, air and oxygen-enriched air.

Preferably, a carbon content in the spent catalyst is 1.0wt% to 3.0wt%.

Preferably, a carbon content in the regenerated catalyst is less than or equal to 0.5wt%.

Preferably, process conditions of the regeneration zone of the regenerator are: an apparent linear velocity of gas of 0.5 m/s to 2.0 m/s, a regeneration temperature of 600°C to 750°C, a regeneration pressure of 100 kPa to 500 kPa, and a bed density of 150 kg/m³ to 700 kg/m³.

Optionally, the apparent linear velocity of gas is independently selected from any value or a value in a range between any two of 0.5 m/s, 0.6 m/s, 0.7 m/s, 0.8 m/s, 0.9 m/s, 1.0 m/s, 1.1 m/s, 1.2 m/s, 1.3 m/s, 1.4 m/s, 1.5 m/s, 1.6 m/s, 1.7 m/s, 1.8 m/s, 1.9 m/s and 2.0 m/s.

Optionally, the regeneration temperature is independently selected from any value or a value in a range between any two of 600°C, 615°C, 630°C, 645°C, 670°C, 685°C, 700°C, 715°C, 730°C, 745°C and 750°C.

Optionally, the regeneration pressure is independently selected from any value or a value in a range between any two of 100 kPa, 125 kPa, 150 kPa, 175 kPa, 200 kPa, 225 kPa, 250 kPa, 275 kPa, 300 kPa, 325 kPa, 350 kPa, 375 kPa, 400 kPa, 425 kPa, 450 kPa, 475 kPa and 500 kPa.

Optionally, the bed density is independently selected from any value or a value in a range between any two of 150 kg/m3, 200 kg/m³, 250 kg/m³, 300 kg/m³, 350 kg/m³, 400 kg/m³, 450 kg/m³, 500 kg/m³, 550 kg/m³, 600 kg/m³, 650 kg/m³ and 700 kg/m³.

Preferably, coke on the spent catalyst reacts with the regeneration gas to generate flue gas, and the flue gas enters a third gas-solid separation zone to remove a regenerated catalyst contained in the flue gas.

Preferably, the flue gas enters the third gas-solid separation zone to remove the regenerated catalyst contained in the flue gas, which specifically comprises that: the flue gas enters a regenerator gas-solid separation device first, and after the regenerated catalyst contained in the flue gas is removed, the flue gas passes through a regenerator gas collection chamber and a flue gas conveying pipe to enter a downstream working section.

Preferably, the regenerated catalyst enters the light hydrocarbon aromatization reactor through a regenerator stripper and a regenerated slide valve.

Preferably, the method further comprises: introducing a riser reactor raw material into an inlet end of a riser reactor of the light hydrocarbon aromatization reactor; and introducing the regenerated catalyst into the riser reactor through a regenerator stripper and a regenerated slide valve, converting the riser reactor raw material into the BTX-containing stream under an action of the regenerated catalyst, and allowing the BTX-containing stream to enter an inner lower portion of a light hydrocarbon aromatization reaction zone in a bed reactor through an outlet end of the riser reactor.

Preferably, the method further comprises: introducing a catalyst into an inlet end of the riser reactor of the light hydrocarbon aromatization reactor, and allowing the catalyst to enter the bed reactor through the riser reactor.

Preferably, the riser reactor raw material comprises water vapor and the low-carbon alkanes separated from the product gas stream.

Preferably, a water vapor content in the riser reactor raw material is 0wt% to 80wt%.

Preferably, process conditions of the riser reactor are: an apparent linear velocity of gas of 3.0 m/s to 10.0 m/s, a temperature of 580°C to 700°C, a pressure of 100 kPa to 500 kPa, and a bed density of 50 kg/m³ to 150 kg/m³.

Optionally, the apparent linear velocity of gas is independently selected from any value or a value in a range between any two of 3.0 m/s, 3.5 m/s, 4.0 m/s, 4.5 m/s, 5.0 m/s, 5.5 m/s, 6.0 m/s, 6.5 m/s, 7.0 m/s, 7.5 m/s, 8.0 m/s, 8.5 m/s, 9.0 m/s, 9.5 m/s and 10.0 m/s.

Optionally, the temperature is independently selected from any value or a value in a range between any two of 580°C, 590°C, 600°C, 610°C, 620°C, 630°C, 640°C, 650°C, 660°C, 670°C, 680°C, 690°C and 700°C.

Optionally, the pressure is independently selected from any value or a value in a range between any two of 100 kPa, 125 kPa, 150 kPa, 175 kPa, 200 kPa, 225 kPa, 250 kPa, 275 kPa, 300 kPa, 325 kPa, 350 kPa, 375 kPa, 400 kPa, 425 kPa, 450 kPa, 475 kPa and 500 kPa.

Optionally, the bed density is independently selected from any value or a value in a range between any two of 50 kg/m3, 60 kg/m³, 70 kg/m³, 80 kg/m³, 90 kg/m³, 100 kg/m³, 110 kg/m³, 120 kg/m³, 130 kg/m³, 140 kg/m³ and 150 kg/m³.

Preferably, the method further comprises: introducing a bed reactor raw material into the light hydrocarbon aromatization reaction zone through a bed reactor distributor to make contact with the catalyst from the riser reactor to generate the light hydrocarbon aromatization product gas.

Optionally, the bed reactor raw material comprises C₄ and C₅ hydrocarbons. The C₄ and C₅ hydrocarbons come from C₄ and C₅ hydrocarbons separated from the product gas stream.

Preferably, the bed reactor raw material comprises C₃, C₄ and C₅ hydrocarbons. The C₃, C₄ and C₅ hydrocarbons come from C₃, C₄ and C₅ hydrocarbons separated from the product gas stream.

Preferably, the BTX-containing stream comprises components of BTX, low-carbon olefins and Hz.

Preferably, the method further comprises:
introducing the spent catalyst in the naphtha and methanol coupled aromatic hydrocarbon preparation reaction zone into the reactor stripper, and allowing the spent catalyst to enter a downstream area through a spent slide valve and a spent agent conveying pipe after stripping; and
preferably, the downstream area is a regenerator.

In the present application, a potential content of aromatic hydrocarbon in the naphtha raw material is 0wt% to 80wt%, and a per-pass conversion rate of the naphtha is 60wt% to 80wt%. By using the device for preparing aromatic hydrocarbons by coupling naphtha and methanol and the method for preparing aromatic hydrocarbons by coupling naphtha and methanol based on the device according to the present application, the unconverted naphtha is separated from the product gas and then returns to the naphtha and methanol coupled aromatic hydrocarbon preparation reactor as a raw material, a part of low-carbon alkanes are separated from the product gas and then return to the riser reactor in the light hydrocarbon aromatization reactor as raw materials, the C₃, C₄ and C₅ hydrocarbons are separated from the product gas and then return to the bed reactor in the light hydrocarbon aromatization reactor as raw materials, and the finally obtained product is composed of: 60wt% to 73wt% BTX, 9wt% to 16wt% low-carbon olefin, 3wt% to 6wt% hydrogen, 3wt% to 8wt% low-carbon alkane, 4wt% to 6wt% combustible gas, 4wt% to 8wt% heavy aromatic hydrocarbon, and 0.5wt% to 1wt% coke. The content of the p-xylene in the mixed xylene in the product is 60wt% to 75wt%.

The present application can achieve the beneficial effects as follows.
1) According to the present application, the linear-chain and branched-chain aliphatic hydrocarbons can be efficiently converted into aromatic hydrocarbons in a highly selective mode, the raw materials have a wide application range, and aromatic hydrocarbons may be prepared from the naphtha with a low potential content of aromatic hydrocarbons.
2) According to the present application, the aromatization of low-carbon alkanes and C₄ and C₅ hydrocarbons is achieved by the light hydrocarbon aromatization reactor and the metal molecular sieve bifunctional catalyst, so that a yield of aromatic hydrocarbons of a technology for preparing aromatic hydrocarbons with naphtha is greatly increased.
3) According to the present application, the device for preparing aromatic hydrocarbons by coupling naphtha and methanol is provided with the naphtha and methanol coupled aromatic hydrocarbon preparation reactor, which comprises a plurality of sub-distributors, naphtha enters the naphtha and methanol coupled aromatic hydrocarbon preparation reaction zone through the 1^{st} sub-distributor, and methanol enters the naphtha and methanol coupled aromatic hydrocarbon preparation reaction zone through the 2^{nd} sub-distributor to the n^{th} sub-distributor respectively. The naphtha and methanol coupled aromatic hydrocarbon preparation reactor is a fluidized bed reactor suitable for a cascade reaction, naphtha is converted into benzene and toluene in a lower portion of the naphtha and methanol coupled aromatic hydrocarbon preparation reaction zone, and then flows upwards to reach a middle portion and an upper portion of the naphtha and methanol coupled aromatic hydrocarbon preparation reaction zone, and benzene and toluene are subjected to the methylation reaction with methanol to further produce the p-xylene, so as to increase the yield of p-xylene. The high-temperature catalyst from the light hydrocarbon aromatization reactor directly enters the lower portion of the naphtha and methanol coupled aromatic hydrocarbon preparation reaction zone, which is beneficial for providing heat required for a reaction of converting naphtha into aromatic hydrocarbons, so as to improve a conversion rate of naphtha. Methanol directly enters the middle portion and the upper portion of the naphtha and methanol coupled aromatic hydrocarbon preparation reaction zone, which effectively reduces residence time of p-xylene in the reaction zone, inhibits an isomerization reaction of p-xylene, increases the content (up to 75wt% under optimal technological conditions) of p-xylene in the xylene, and greatly reduces the energy consumption for separation of p-xylene at the same time. In short, in the naphtha and methanol coupled aromatic hydrocarbon preparation reactor, the naphtha raw material flows from bottom to top, and in a process of converting naphtha into aromatic hydrocarbons, the methylation raw material (methanol) is added step by step to control a process of the cascade reaction (naphtha → benzene and toluene → p-xylene), so as to increase the yield of the p-xylene.
4) The aromatization reaction of the naphtha is a strong endothermic reaction, and 1.1 MJ to 1.6 MJ of heat needs to be absorbed to convert 1 kg of naphtha into aromatic hydrocarbons. The methylation reaction of methanol and aromatic hydrocarbons is a strong exothermic reaction, and more than 2.0 MJ of heat may be released in the conversion of 1 kg of methanol into the methyl on aromatic hydrocarbons. Therefore, the preparation of p-xylene from benzene, toluene and methanol may also provide heat in situ for a naphtha and methanol coupled aromatic hydrocarbon preparation reaction, so that self-heating balance is achieved.
5) According to the present application, the device for preparing aromatic hydrocarbons by coupling naphtha and methanol is provided with an independent light hydrocarbon aromatization reactor. Low-carbon alkanes are very stable and need a high reaction temperature, a temperature of the light hydrocarbon aromatization reactor is higher than that of the naphtha and methanol coupled aromatic hydrocarbon preparation reactor, and low-carbon alkanes and C₄ and C₅ hydrocarbons are subjected to the aromatization reaction in the independent light hydrocarbon aromatization reactor, so that the reaction rate and the yield of aromatic hydrocarbons are increased.

### BRIEF DESCRIPTION OF THE DRAWINGS

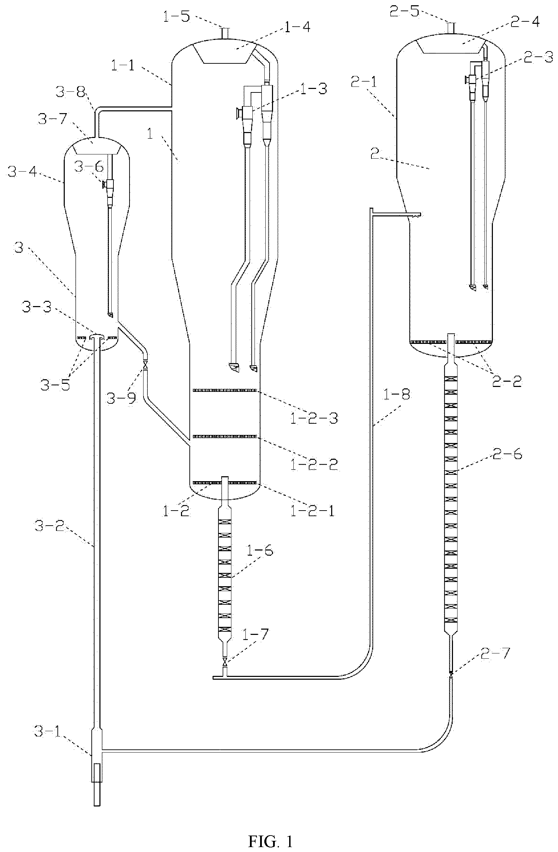

FIG. 1 is a schematic diagram of a device for preparing aromatic hydrocarbons by coupling naphtha and methanol in one embodiment of the present application.

### List of parts and reference symbols:

1 refers to naphtha and methanol coupled aromatic hydrocarbon preparation reactor;
1-1 refers to naphtha and methanol coupled aromatic hydrocarbon preparation reactor shell;
1-2 refers to naphtha and methanol coupled aromatic hydrocarbon preparation reactor distributor;
1-3 refers to gas-solid separation device I; 1-4 refers to gas collection chamber I; 1-5 refers to product gas conveying pipe I;
1-6 refers to reactor stripper; 1-7 refers to spent slide valve; 1-8 refers to spent agent conveying pipe;
1-2-1 refers to 1^{st} sub-distributor; 1-2-2 refers to 2^{nd} sub-distributor; 1-2-3 refers to 3^{rd} sub-distributor;
2 refers to regenerator;
2-1 refers to regenerator shell; 2-2 refers to regenerator distributor;
2-3 refers to regenerator gas-solid separation device; 2-4 refers to regenerator gas collection chamber; 2-5 refers to flue gas conveying pipe;
2-6 refers to regenerator stripper; 2-7 refers to regenerated slide valve;
3 refers to light hydrocarbon aromatization reactor;
3-1 refers to inlet end of riser reactor; 3-2 refers to middle portion of riser reactor;
3-3 refers to outlet end of riser reactor; 3-4 refers to bed reactor shell;
3-5 refers to bed reactor distributor; 3-6 refers to gas-solid separation device II;
3-7 refers to gas collection chamber II; 3-8 refers to product gas conveying pipe II; 3-9 refers to light hydrocarbon aromatization slide valve.

### DETAILED DESCRIPTION

The present application is described in detail hereinafter with reference to examples, but the present application is not limited to these examples.

The present application provides a device for preparing aromatic hydrocarbons by coupling naphtha and methanol, which comprises a light hydrocarbon aromatization reactor and a naphtha and methanol coupled aromatic hydrocarbon preparation reactor; wherein,
the light hydrocarbon aromatization reactor is used for introducing raw materials and a high-temperature catalyst; and at least one outlet of the light hydrocarbon aromatization reactor is connected to the naphtha and methanol coupled aromatic hydrocarbon preparation reactor for conveying the catalyst and generated light hydrocarbon aromatization product gas to the naphtha and methanol coupled aromatic hydrocarbon preparation reactor; and
the naphtha and methanol coupled aromatic hydrocarbon preparation reactor is used for introducing the naphtha and the methanol and allowing the naphtha to make contact with the catalyst from the light hydrocarbon aromatization reactor to generate a BTX-containing product gas stream after reaction; and subjecting the methanol to a methylation reaction with benzene and toluene in the product gas stream to generate p-xylene.

The naphtha in the present application comprises components of C₄-C₁₂ linear-chain and branched-chain aliphatic hydrocarbons, naphthenic hydrocarbons and aromatic hydrocarbons.

The BTX in the present application is aromatic hydrocarbons, and refers to benzene, toluene and xylene.

In a preferred embodiment, the device further comprises a regenerator, and at least one inlet of the light hydrocarbon aromatization reactor is connected to the regenerator for acquiring a high-temperature regenerated catalyst generated by the regenerator.

With reference to FIG. 1, the device for preparing aromatic hydrocarbons by coupling naphtha and methanol in a preferred embodiment of the present application comprises a naphtha and methanol coupled aromatic hydrocarbon preparation reactor 1, a regenerator 2 and a light hydrocarbon aromatization reactor 3.

The naphtha and methanol coupled aromatic hydrocarbon preparation reactor 1 comprises a naphtha and methanol coupled aromatic hydrocarbon preparation reactor shell 1-1, a naphtha and methanol coupled aromatic hydrocarbon preparation reactor distributor 1-2, a gas-solid separation device I 1-3, a gas collection chamber I 1-4, a product gas conveying pipe I 1-5, a reactor stripper 1-6, a spent slide valve 1-7 and a spent agent conveying pipe 1-8.

The naphtha and methanol coupled aromatic hydrocarbon preparation reactor distributor 1-2 comprises a 1^{st} sub-distributor 1-2-1, a 2^{nd} sub-distributor 1-2-2 and a 3^{rd} sub-distributor 1-2-3.

The naphtha and methanol coupled aromatic hydrocarbon preparation reactor 1 comprises the naphtha and methanol coupled aromatic hydrocarbon preparation reactor shell 1-1, the naphtha and methanol coupled aromatic hydrocarbon preparation reactor shell 1-1 comprises a naphtha and methanol coupled aromatic hydrocarbon preparation reactor upper shell and a naphtha and methanol coupled aromatic hydrocarbon preparation reactor lower shell, a first gas-solid separation zone is enclosed by the naphtha and methanol coupled aromatic hydrocarbon preparation reactor upper shell, a naphtha and methanol coupled aromatic hydrocarbon preparation reaction zone is enclosed by the naphtha and methanol coupled aromatic hydrocarbon preparation reactor lower shell, and an outlet of the light hydrocarbon aromatization reactor 3 is provided in the naphtha and methanol coupled aromatic hydrocarbon preparation reactor shell 1-1.

The naphtha and methanol coupled aromatic hydrocarbon preparation reactor distributor 1-2 is provided in the naphtha and methanol coupled aromatic hydrocarbon preparation reaction zone. The naphtha and methanol coupled aromatic hydrocarbon preparation reactor distributor 1-2 comprises 3 sub-distributors, which are sequentially the 1^{st} sub-distributor 1-2-1 to the 3^{rd} sub-distributor 1-2-3 from bottom to top. The 1^{st} sub-distributor 1-2-1 is used for introducing a naphtha raw material; and the 2^{nd} sub-distributor 1-2-2 to the 3^{rd} sub-distributor 1-2-3 are used for introducing a methanol raw material.

The gas-solid separation device I 1-3 and the gas collection chamber I 1-4 are also provided in the naphtha and methanol coupled aromatic hydrocarbon preparation reactor shell 1-1; the gas collection chamber I 1-4 is located on an inner top portion of the naphtha and methanol coupled aromatic hydrocarbon preparation reactor shell; a gas outlet of the gas-solid separation device I 1-3 is communicated with the gas collection chamber I 1-4; the gas collection chamber I 1-4 is communicated with the product gas conveying pipe I 1-5; and a catalyst outlet end of the gas-solid separation device 11-3 is located above an opening end of an inlet pipe of the reactor stripper 1-6.

The reactor stripper 1-6 is provided below the naphtha and methanol coupled aromatic hydrocarbon preparation reaction zone; an inlet of the reactor stripper 1-6 is located inside the naphtha and methanol coupled aromatic hydrocarbon preparation reactor shell 1-1; an outlet of the reactor stripper 1-6 is located outside the naphtha and methanol coupled aromatic hydrocarbon preparation reactor shell 1-1 and connected with the spent slide valve 1-7; and an opening end of the inlet of the reactor stripper 1-6 is located above the 1^{st} sub-distributor 1-2-1.

The spent slide valve 1-7 is provided below the reactor stripper 1-6; and an inlet of the spent slide valve 1-7 is connected to the outlet of the reactor stripper 1-6, an outlet of the spent slide valve 1-7 is connected to an inlet of the spent agent conveying pipe 1-8, and an outlet of the spent agent conveying pipe 1-8 is connected to a regenerator shell 2-1.

The spent slide valve 1-7 is used for controlling a circulating amount of a spent catalyst.

In a preferred embodiment, the gas-solid separation device I 1-3 is one or more groups of gas-solid cyclone separators, and each group of gas-solid cyclone separators comprises a first-stage gas-solid cyclone separator and a second-stage gas-solid cyclone separator.

The regenerator 2 comprises the regenerator shell 2-1, a regenerator distributor 2-2, a regenerator gas-solid separation device 2-3, a regenerator gas collection chamber 2-4, a flue gas conveying pipe 2-5, a regenerator stripper 2-6 and a regenerated slide valve 2-7.

The regenerator shell 2-1 comprises a regenerator upper shell and a regenerator lower shell, a third gas-solid separation zone is enclosed by the regenerator upper shell, and a regeneration zone is enclosed by the regenerator lower shell; and the outlet of the spent agent conveying pipe 1-8 is provided in the regenerator shell 2-1.

The regenerator distributor 2-2 is provided on a lower portion of the regeneration zone, and the regenerator distributor 2-2 is used for introducing regeneration gas.

The regenerator gas-solid separation device 2-3 and the regenerator gas collection chamber 2-4 are also provided in the regenerator shell 2-1; the regenerator gas collection chamber 2-4 is located on an inner top portion of the regenerator shell 2-1; a gas outlet of the regenerator gas-solid separation device 2-3 is communicated with the regenerator gas collection chamber 2-4; the regenerator gas collection chamber 2-4 is communicated with the flue gas conveying pipe 2-5; and a catalyst outlet end of the regenerator gas-solid separation device 2-3 is located above an opening end of an inlet pipe of the regenerator stripper 2-6.

The regenerator stripper 2-6 is provided below the regeneration zone; an inlet of the regenerator stripper 2-6 is located inside the regenerator shell 2-1; an outlet of the regenerator stripper 2-6 is located outside the regenerator shell 2-1 and connected with the regenerated slide valve 2-7; and an opening end of the inlet of the regenerator stripper 2-6 is located above the regenerator distributor 2-2.

The regenerated slide valve 2-7 is provided below the regenerator stripper 2-6; and an inlet of the regenerated slide valve 2-7 is connected to the outlet of the regenerator stripper 2-6.

The regenerated slide valve 2-7 is used for controlling a circulating amount of a regenerated catalyst.

In a preferred embodiment, the regenerator gas-solid separation device 2-3 is one or more groups of gas-solid cyclone separators, and each group of gas-solid cyclone separators comprises a first-stage gas-solid cyclone separator and a second-stage gas-solid cyclone separator.

The light hydrocarbon aromatization reactor comprises an inlet end 3-1 of a riser reactor, a middle portion 3-2 of the riser reactor, an outlet end 3-3 of the riser reactor, a bed reactor shell 3-4, a bed reactor distributor 3-5, a gas-solid separation device II 3-6, a gas collection chamber II 3-7, a product gas conveying pipe II 3-8 and a light hydrocarbon aromatization slide valve 3-9.

The bed reactor shell 3-4 comprises a bed reactor upper shell and a bed reactor lower shell, a second gas-solid separation zone is enclosed by the bed reactor upper shell, and a light hydrocarbon aromatization reaction zone is enclosed by the bed reactor lower shell; the bed reactor distributor 3-5 is provided on an inner lower portion of the light hydrocarbon aromatization reaction zone; the light hydrocarbon aromatization slide valve 3-9 is also provided outside the light hydrocarbon aromatization reaction zone; an upper section of the riser reactor penetrates through a bottom portion of the bed reactor to be inserted into the bed reactor in an axial direction; and the outlet end 3-3 of the riser reactor is located on the inner lower portion of the light hydrocarbon aromatization reaction zone.

The light hydrocarbon aromatization slide valve 3-9 is used for conveying the catalyst to the next reactor, such as the naphtha and methanol coupled aromatic hydrocarbon preparation reactor 1.

The second gas-solid separation zone is provided with the gas-solid separation device II 3-6 and the gas collection chamber II 3-7; a gas outlet of the gas-solid separation device II 3-6 is communicated with the gas collection chamber II 3-7; a catalyst outlet of the gas-solid separation device II 3-6 is located in the light hydrocarbon aromatization reaction zone; and the gas collection chamber II 3-7 is communicated with the product gas conveying pipe II 3-8 located outside the bed reactor.

In a preferred embodiment, the gas-solid separation device II 3-6 is a gas-solid cyclone separator.

In a preferred embodiment, the gas collection chamber II 3-7 is provided on an inner top portion of the bed reactor; and a catalyst outlet of the gas-solid cyclone separator 3-7 of the bed reactor is located above the outlet end 3-3 of the riser reactor.

In a preferred embodiment, the bed reactor distributor 3-5 is used for introducing a bed reactor raw material.

In a preferred embodiment, an inlet end 3-1 of the riser reactor is also used for introducing the catalyst and a riser reactor raw material.

An inlet of the light hydrocarbon aromatization reactor 3 is connected to the regenerator 2, and an outlet of the light hydrocarbon aromatization reactor 3 is connected to the naphtha and methanol coupled aromatic hydrocarbon preparation reactor 1.

In a preferred embodiment, an inlet end 3-1 of the riser reactor is connected to the regenerated slide valve 2-7 through a pipeline, and the light hydrocarbon aromatization slide valve 3-9 is connected to the naphtha and methanol coupled aromatic hydrocarbon preparation reactor shell 1-1 through a pipeline, and located between the 1^{st} sub-distributor 1-2-1 and the 2^{nd} sub-distributor 1-2-2.

In a preferred embodiment, the product gas conveying pipe II 3-8 is connected to the naphtha and methanol coupled aromatic hydrocarbon preparation reactor shell 1-1.

The present application further provides a method for preparing aromatic hydrocarbons by coupling naphtha and methanol, which comprises: preparing aromatic hydrocarbons by using the device for preparing aromatic hydrocarbons by coupling naphtha and methanol above and a catalyst.

The catalyst is a metal molecular sieve bifunctional catalyst. A metal modified HZSM-5 zeolite molecular sieve is used in Examples 1 to 5.

Metal for the metal modification is selected from at least one of La, Zn, Ga, Fe, Mo and Cr.

A method for the metal modification comprises: placing the HZSM-5 zeolite molecular sieve in a metal salt solution, soaking, drying and roasting to obtain the metal modified HZSM-5 zeolite molecular sieve.

In a preferred embodiment, the method comprises the following steps.
a) Naphtha enters the naphtha and methanol coupled aromatic hydrocarbon preparation reaction zone of the naphtha and methanol coupled aromatic hydrocarbon preparation reactor 1 through the 1^{st} sub-distributor 1-2-1 of the naphtha and methanol coupled aromatic hydrocarbon preparation reactor distributor 1-2, and makes contact with the catalyst from the light hydrocarbon aromatization reactor 3 to generate a product gas stream containing BTX, low-carbon olefins, hydrogen, low-carbon alkanes, combustible gas, heavy aromatic hydrocarbons and unconverted naphtha; methanol enters the naphtha and methanol coupled aromatic hydrocarbon preparation reaction zone through the 2^{nd} sub-distributor 1-2-2 to the 3^{rd} sub-distributor 1-2-3 of the naphtha and methanol coupled aromatic hydrocarbon preparation reactor distributor 1-2 respectively, and is subjected to a methylation reaction with benzene and toluene in the product gas stream to generate p-xylene; the catalyst from the light hydrocarbon aromatization reactor 3 is coked and converted into a spent catalyst in the naphtha and methanol coupled aromatic hydrocarbon preparation reaction zone; the product gas stream enters the gas-solid separation device I 1-3 to remove the spent catalyst contained in the product gas stream, then enters the gas collection chamber I 1-4, and enters a downstream working section through the product gas conveying pipe I 1-5; and the spent catalyst in the naphtha and methanol coupled aromatic hydrocarbon preparation reaction zone enters the reactor stripper 1-6 from the opening end of the inlet pipe of the reactor stripper 1-6 for stripping, and then enters the regenerator 2 through the spent slide valve 1-7 and the spent agent conveying pipe 1-8 after stripping.
b) The regeneration gas is introduced into the regeneration zone of the regenerator 2 through the regenerator distributor 2-2 and makes contact with the spent catalyst, coke on the spent catalyst reacts with the regeneration gas to generate flue gas, and meanwhile, the spent catalyst is converted into the regenerated catalyst; the flue gas enters the regenerator gas-solid separation device 2-3 to remove the regenerated catalyst contained in the flue gas, then enters the regenerator gas collection chamber 2-4, and enters the downstream working section through the flue gas conveying pipe 2-5; and the regenerated catalyst enters the light hydrocarbon aromatization reactor 3 through the regenerator stripper 2-6 and the regenerated slide valve 2-7 sequentially.
c) The riser reactor raw material is introduced into the riser reactor through the inlet end 3-1 of the riser reactor, and makes contact and reacts with the regenerated catalyst from the regenerator, and the riser reactor raw material is converted into a stream containing components such as BTX, low-carbon olefins and H₂ under an action of the catalyst, and then enters the inner lower portion of the light hydrocarbon aromatization reaction zone in the bed reactor through the outlet end 3-3 of the riser reactor; the bed reactor raw material is introduced into the light hydrocarbon aromatization reaction zone through the bed reactor distributor 3-5, and makes contact with the catalyst from the riser reactor to generate a light hydrocarbon aromatization product gas containing components such as BTX, low-carbon olefins and H₂; the light hydrocarbon aromatization product gas enters the gas-solid separation device II 3-6 to remove the catalyst contained in the light hydrocarbon aromatization product gas, then enters the gas collection chamber II 3-7, and enters the first gas-solid separation zone through the product gas conveying pipe II 3-8; and the catalyst in the light hydrocarbon aromatization reaction zone enters the naphtha and methanol coupled aromatic hydrocarbon preparation reactor 1 through the light hydrocarbon aromatization slide valve 3-9.

The low-carbon olefin refers to ethylene and propylene.

The low-carbon alkane refers to ethane and propane.

The combustible gas comprises methane, CO, and the like.

The heavy aromatic hydrocarbon refers to an aromatic hydrocarbon with a number of carbon atoms in a molecule greater than or equal to 9.

In a preferred embodiment, the naphtha is selected from at least one of direct coal liquefaction naphtha, indirect coal liquefaction naphtha, straight-run naphtha and hydrocracking naphtha.

In a preferred embodiment, the naphtha also contains unconverted naphtha separated from the product gas stream.

In a preferred embodiment, a carbon content in the spent catalyst is 1.0wt% to 3.0wt%.

In a preferred embodiment, process conditions of the naphtha and methanol coupled aromatic hydrocarbon preparation reaction zone are: an apparent linear velocity of gas of 0.5 m/s to 2.0 m/s, a reaction temperature of 500°C to 600°C, a reaction pressure of 100 kPa to 500 kPa, and a bed density of 150 kg/m³ to 700 kg/m³.

Optionally, the apparent linear velocity of gas of the naphtha and methanol coupled aromatic hydrocarbon preparation reaction zone is independently selected from any value or a value in a range between any two of 0.5 m/s, 0.6 m/s, 0.7 m/s, 0.8 m/s, 0.9 m/s, 1.0 m/s, 1.1 m/s, 1.2 m/s, 1.3 m/s, 1.4 m/s, 1.5 m/s, 1.6 m/s, 1.7 m/s, 1.8 m/s, 1.9 m/s and 2.0 m/s.

Optionally, the reaction temperature of the naphtha and methanol coupled aromatic hydrocarbon preparation reaction zone is independently selected from any value or a value in a range between any two of 500°C, 520°C, 530°C, 540°C, 550°C, 560°C, 570°C, 580°C, 590°C and 600°C.

Optionally, the reaction pressure of the naphtha and methanol coupled aromatic hydrocarbon preparation reaction zone is independently selected from any value or a value in a range between any two of 100 kPa, 125 kPa, 150 kPa, 175 kPa, 200 kPa, 225 kPa, 250 kPa, 275 kPa, 300 kPa, 325 kPa, 350 kPa, 375 kPa, 400 kPa, 425 kPa, 450 kPa, 475 kPa and 500 kPa.

Optionally, the bed density of the naphtha and methanol coupled aromatic hydrocarbon preparation reaction zone is independently selected from any value or a value in a range between any two of 150 kg/m³, 200 kg/m³, 250 kg/m³, 300 kg/m³, 350 kg/m³, 400 kg/m³, 450 kg/m³, 500 kg/m³, 550 kg/m³, 600 kg/m³, 650 kg/m³ and 700 kg/m³.

In a preferred embodiment, a carbon content in the regenerated catalyst is less than or equal to 0.5wt%.

In a preferred embodiment, the regeneration gas is selected from at least one of oxygen, air and oxygen-enriched air.

In a preferred embodiment, process conditions of the regeneration zone are: an apparent linear velocity of gas of 0.5 m/s to 2.0 m/s, a regeneration temperature of 600°C to 750°C, a regeneration pressure of 100 kPa to 500 kPa, and a bed density of 150 kg/m³ to 700 kg/m³.

Optionally, the apparent linear velocity of gas of the regeneration zone is independently selected from any value or a value in a range between any two of 0.5 m/s, 0.6 m/s, 0.7 m/s, 0.8 m/s, 0.9 m/s, 1.0 m/s, 1.1 m/s, 1.2 m/s, 1.3 m/s, 1.4 m/s, 1.5 m/s, 1.6 m/s, 1.7 m/s, 1.8 m/s, 1.9 m/s and 2.0 m/s.

Optionally, the regeneration temperature of the regeneration zone is independently selected from any value or a value in a range between any two of 600°C, 615°C, 630°C, 645°C, 670°C, 685°C, 700°C, 715°C, 730°C, 745°C and 750°C.

Optionally, the regeneration pressure of the regeneration zone is independently selected from any value or a value in a range between any two of 100 kPa, 125 kPa, 150 kPa, 175 kPa, 200 kPa, 225 kPa, 250 kPa, 275 kPa, 300 kPa, 325 kPa, 350 kPa, 375 kPa, 400 kPa, 425 kPa, 450 kPa, 475 kPa and 500 kPa.

Optionally, the bed density of the regeneration zone is independently selected from any value or a value in a range between any two of 150 kg/m³, 200 kg/m³, 250 kg/m³, 300 kg/m³, 350 kg/m³, 400 kg/m³, 450 kg/m³, 500 kg/m³, 550 kg/m³, 600 kg/m³, 650 kg/m³ and 700 kg/m³.

In a preferred embodiment, the riser reactor raw material comprises water vapor and the low-carbon alkanes separated from the product gas stream.

In a preferred embodiment, a water vapor content in the riser reactor raw material is 0wt% to 80wt%.

In a preferred embodiment, process conditions of the riser reactor are: an apparent linear velocity of gas of 3.0 m/s to 10.0 m/s, a temperature of 580°C to 700°C, a pressure of 100 kPa to 500 kPa, and a bed density of 50 kg/m³ to 150 kg/m³.

Optionally, the apparent linear velocity of gas of the riser reactor is independently selected from any value or a value in a range between any two of 3.0 m/s, 3.5 m/s, 4.0 m/s, 4.5 m/s, 5.0 m/s, 5.5 m/s, 6.0 m/s, 6.5 m/s, 7.0 m/s, 7.5 m/s, 8.0 m/s, 8.5 m/s, 9.0 m/s, 9.5 m/s and 10.0 m/s.

Optionally, the temperature of the riser reactor is independently selected from any value or a value in a range between any two of 580°C, 590°C, 600°C, 610°C, 620°C, 630°C, 640°C, 650°C, 660°C, 670°C, 680°C, 690°C and 700°C.

Optionally, the pressure of the riser reactor is independently selected from any value or a value in a range between any two of 100 kPa, 125 kPa, 150 kPa, 175 kPa, 200 kPa, 225 kPa, 250 kPa, 275 kPa, 300 kPa, 325 kPa, 350 kPa, 375 kPa, 400 kPa, 425 kPa, 450 kPa, 475 kPa and 500 kPa.

Optionally, the bed density of the riser reactor is independently selected from any value or a value in a range between any two of 50 kg/m3, 60 kg/m³, 70 kg/m³, 80 kg/m³, 90 kg/m³, 100 kg/m³, 110 kg/m³, 120 kg/m³, 130 kg/m³, 140 kg/m³ and 150kg/m³.

The bed reactor raw material comprises C₄ and C₅ hydrocarbons.

In a preferred embodiment, the bed reactor raw material comprises C₃, C₄ and C₅ hydrocarbons.

In a preferred embodiment, the C₃, C₄ and C₅ hydrocarbons come from C₃, C₄ and C₅ hydrocarbons separated from the product gas stream.

The C₃, C₄ and C₅ hydrocarbons refer to hydrocarbons with 3, 4 and 5 C atoms.

In a preferred embodiment, process conditions of the light hydrocarbon aromatization reaction zone are: an apparent linear velocity of gas of 0.5 m/s to 2.0 m/s, a reaction temperature of 550°C to 665°C, a reaction pressure of 100 kPa to 500 kPa, and a bed density of 150 kg/m³ to 700 kg/m³.

Optionally, the apparent linear velocity of gas of the light hydrocarbon aromatization reaction zone is independently selected from any value or a value in a range between any two of 0.5 m/s, 0.6 m/s, 0.7 m/s, 0.8 m/s, 0.9 m/s, 1.0 m/s, 1.1 m/s, 1.2 m/s, 1.3 m/s, 1.4 m/s, 1.5 m/s, 1.6 m/s, 1.7 m/s, 1.8 m/s, 1.9 m/s and 2.0 m/s.

Optionally, the reaction temperature of the light hydrocarbon aromatization reaction zone is independently selected from any value or a value in a range between any two of 550°C, 560°C, 570°C, 580°C, 590°C, 600°C, 610°C, 620°C, 630°C, 640°C, 650°C, 660°C and 665°C.

Optionally, the reaction pressure of the light hydrocarbon aromatization reaction zone is independently selected from any value or a value in a range between any two of 100 kPa, 125 kPa, 150 kPa, 175 kPa, 200 kPa, 225 kPa, 250 kPa, 275 kPa, 300 kPa, 325 kPa, 350 kPa, 375 kPa, 400 kPa, 425 kPa, 450 kPa, 475 kPa and 500 kPa.

Optionally, the bed density of the light hydrocarbon aromatization reaction zone is independently selected from any value or a value in a range between any two of 150 kg/m³, 200 kg/m³, 250 kg/m³, 300 kg/m³, 350 kg/m³, 400 kg/m³, 450 kg/m³, 500 kg/m³, 550 kg/m³, 600 kg/m³, 650 kg/m³ and 700 kg/m³.

In the embodiments of the present application, a potential content of aromatic hydrocarbon in the naphtha raw material is 0wt% to 80wt%, a per-pass conversion rate of naphtha is 60wt% to 80wt%, and a per-pass conversion rate of methanol is about 100wt%. The unconverted naphtha is separated from the product gas and then returns to the naphtha and methanol coupled aromatic hydrocarbon preparation reactor as a raw material, a part of low-carbon alkanes are separated from the product gas and then return to the riser reactor in the light hydrocarbon aromatization reactor as raw materials, the C₃, C₄ and C₅ hydrocarbons are separated from the product gas and then return to the bed reactor in the light hydrocarbon aromatization reactor as raw materials, and the finally obtained product is composed of: 60wt% to 73wt% BTX, 9wt% to 16wt% low-carbon olefin, 3wt% to 6wt% hydrogen, 3wt% to 8wt% low-carbon alkane, 4wt% to 6wt% combustible gas, 4wt% to 8wt% heavy aromatic hydrocarbon, and 0.5wt% to 1wt% coke. The content of p-xylene in the mixed xylene in the product is 60wt% to 75wt%.

### Example 1

A device shown in FIG. 1 is adopted in this embodiment.

In this embodiment, a naphtha raw material entering a naphtha and methanol coupled aromatic hydrocarbon preparation reactor is direct coal liquefaction naphtha, and a potential content of aromatic hydrocarbon in the naphtha is 78wt%.

Process conditions of a naphtha and methanol coupled aromatic hydrocarbon preparation reaction zone of the naphtha and methanol coupled aromatic hydrocarbon preparation reactor are: an apparent linear velocity of gas of 0.5 m/s, a reaction temperature of 600°C, a reaction pressure of 100 kPa, and a bed density of 700 kg/m³.

Regeneration gas is air.

Process conditions of a regeneration zone of a regenerator are: an apparent linear velocity of gas of 0.5 m/s, a regeneration temperature of 745°C, a regeneration pressure of 100 kPa, and a bed density of 700 kg/m³.

A riser reactor raw material is a low-carbon alkane separated from a product gas stream.

Process conditions of a riser reactor are: an apparent linear velocity of gas of 3.0 m/s, a temperature of 690°C, a pressure of 100 kPa, and a bed density of 150 kg/m³.

A bed reactor raw material is unconverted naphtha separated from the product gas stream, and the unconverted naphtha comprises main components of C₄-C₁₂ linear-chain and branched-chain aliphatic hydrocarbons and naphthenic hydrocarbons.

Process conditions of a light hydrocarbon aromatization reaction zone are: an apparent linear velocity of gas of 0.5 m/s, a reaction temperature of 665°C, a reaction pressure of 100 kPa, and a bed density of 700 kg/m³.

A carbon content in a spent catalyst is 1.0wt%, and a carbon content in a regenerated catalyst is 0.2wt%.

The naphtha raw material entering the naphtha and methanol coupled aromatic hydrocarbon preparation reactor has a per-pass conversion rate of 61wt%.

The product is composed of 73wt% BTX, 9wt% low-carbon olefin, 3wt% hydrogen, 3wt% low-carbon alkane, 5wt% combustible gas, 6.5wt% heavy aromatic hydrocarbon and 0.5wt% coke. A content of p-xylene in the mixed xylene in the product is 60wt%.

### Example 2

A device shown in FIG. 1 is adopted in this embodiment.

In this embodiment, a naphtha raw material entering a naphtha and methanol coupled aromatic hydrocarbon preparation reactor is indirect coal liquefaction naphtha, and a potential content of aromatic hydrocarbon in the naphtha is 0.1wt%. The naphtha raw material entering the naphtha and methanol coupled aromatic hydrocarbon preparation reactor further comprises unconverted naphtha separated from a product gas stream.

Process conditions of a naphtha and methanol coupled aromatic hydrocarbon preparation reaction zone of the naphtha and methanol coupled aromatic hydrocarbon preparation reactor are: an apparent linear velocity of gas of 2.0m/s, a reaction temperature of 510°C, a reaction pressure of 500 kPa, and a bed density of 150 kg/m³.

Regeneration gas is oxygen.

Process conditions of a regeneration zone of a regenerator are: an apparent linear velocity of gas of 2.0 m/s, a regeneration temperature of 610°C, a regeneration pressure of 500 kPa, and a bed density of 150 kg/m³.

A riser reactor raw material comprises water vapor and low-carbon alkanes separated from the product gas stream, wherein a water vapor content in the riser reactor raw material is 80wt%.

Process conditions of a riser reactor are: an apparent linear velocity of gas of 10.0 m/s, a temperature of 580°C, a pressure of 500 kPa, and a bed density of 50 kg/m³.

A bed reactor raw material is C₃, C₄ and C₅ hydrocarbons separated from the product gas stream.

Process conditions of a light hydrocarbon aromatization reaction zone are: an apparent linear velocity of gas of 2.0 m/s, a reaction temperature of 550°C, a reaction pressure of 500 kPa, and a bed density of 150 kg/m³.

A carbon content in a spent catalyst is 3.0wt%, and a carbon content in a regenerated catalyst is 0.1wt%.

The naphtha raw material entering the naphtha and methanol coupled aromatic hydrocarbon preparation reactor has a per-pass conversion rate of 66wt%.

The product is composed of 65wt% BTX, 13wt% low-carbon olefin, 5wt% hydrogen, 3.2wt% low-carbon alkane, 5wt% combustible gas, 8wt% heavy aromatic hydrocarbon and 0.8wt% coke. A content of p-xylene in the mixed xylene in the product is 66wt%.

### Example 3

A device shown in FIG. 1 is adopted in this embodiment.

In this embodiment, a naphtha raw material entering a naphtha and methanol coupled aromatic hydrocarbon preparation reactor is indirect coal liquefaction naphtha, and a potential content of aromatic hydrocarbon in the naphtha is 3wt%. The naphtha raw material entering the naphtha and methanol coupled aromatic hydrocarbon preparation reactor further comprises unconverted naphtha separated from a product gas stream.

Process conditions of a naphtha and methanol coupled aromatic hydrocarbon preparation reaction zone of the naphtha and methanol coupled aromatic hydrocarbon preparation reactor are: an apparent linear velocity of gas of 1.2 m/s, a reaction temperature of 550°C, a reaction pressure of 120 kPa, and a bed density of 260 kg/m³.

Regeneration gas is oxygen-enriched air.

Process conditions of a regeneration zone of a regenerator are: an apparent linear velocity of gas of 1.2 m/s, a regeneration temperature of 650°C, a regeneration pressure of 120 kPa, and a bed density of 260 kg/m³.

A riser reactor raw material comprises water vapor and low-carbon alkanes separated from the product gas stream, wherein a water vapor content in the riser reactor raw material is 25wt%.

Process conditions of a riser reactor are: an apparent linear velocity of gas of 7.0 m/s, a temperature of 630°C, a pressure of 120 kPa, and a bed density of 80 kg/m³.

A bed reactor raw material is C₄ and C₅ hydrocarbons separated from the product gas stream.

Process conditions of a light hydrocarbon aromatization reaction zone are: an apparent linear velocity of gas of 1.2 m/s, a reaction temperature of 580°C, a reaction pressure of 120 kPa, and a bed density of 260 kg/m³.

A carbon content in a spent catalyst is 2.2wt%, and a carbon content in a regenerated catalyst is 0.3wt%.

The naphtha raw material entering the naphtha and methanol coupled aromatic hydrocarbon preparation reactor has a per-pass conversion rate of 80wt%.

The product is composed of 60wt% BTX, 16wt% low-carbon olefin, 6wt% hydrogen, 8wt% low-carbon alkane, 4.5wt% combustible gas, 5wt% heavy aromatic hydrocarbon and 0.5wt% coke. A content of p-xylene in the mixed xylene in the product is 75wt%.

### Example 4

A device shown in FIG. 1 is adopted in this embodiment.

In this embodiment, a naphtha raw material entering a naphtha and methanol coupled aromatic hydrocarbon preparation reactor is straight-run naphtha, and a potential content of aromatic hydrocarbon in the naphtha is 46wt%. The naphtha raw material entering the naphtha and methanol coupled aromatic hydrocarbon preparation reactor further comprises unconverted naphtha separated from a product gas stream.

Process conditions of a naphtha and methanol coupled aromatic hydrocarbon preparation reaction zone of the naphtha and methanol coupled aromatic hydrocarbon preparation reactor are: an apparent linear velocity of gas of 1.8 m/s, a reaction temperature of 590°C, a reaction pressure of 200 kPa, and a bed density of 220 kg/m³.

Regeneration gas is air.

Process conditions of a regeneration zone of a regenerator are: an apparent linear velocity of gas of 1.8m/s, a regeneration temperature of 700°C, a regeneration pressure of 200 kPa, and a bed density of 220 kg/m³.

A riser reactor raw material comprises water vapor and low-carbon alkanes separated from the product gas stream, wherein a water vapor content in the riser reactor raw material is 50wt%.

Process conditions of a riser reactor are: an apparent linear velocity of gas of 5.0 m/s, a temperature of 660°C, a pressure of 200 kPa, and a bed density of 110 kg/m³.

Abed reactor raw material is C₄ and C₅ hydrocarbons separated from the product gas stream.

Process conditions of a light hydrocarbon aromatization reaction zone are: an apparent linear velocity of gas of 1.8 m/s, a reaction temperature of 630°C, a reaction pressure of 200 kPa, and a bed density of 220 kg/m³.

A carbon content in a spent catalyst is 1.7wt%, and a carbon content in a regenerated catalyst is 0.1 wt%.

The naphtha raw material entering the naphtha and methanol coupled aromatic hydrocarbon preparation reactor has a per-pass conversion rate of 78wt%.

The product is composed of 68.1wt% BTX, 12wt% low-carbon olefin, 5wt% hydrogen, 6wt% low-carbon alkane, 4wt% combustible gas, 4wt% heavy aromatic hydrocarbon and 0.9wt% coke. A content of p-xylene in the mixed xylene in the product is 71wt%.

### Example 5

A device shown in FIG. 1 is adopted in this embodiment.

In this embodiment, a naphtha raw material entering a naphtha and methanol coupled aromatic hydrocarbon preparation reactor is hydrocracking naphtha, and a potential content of aromatic hydrocarbon in the naphtha is 64wt%. The naphtha raw material entering the naphtha and methanol coupled aromatic hydrocarbon preparation reactor further comprises unconverted naphtha separated from a product gas stream.

Process conditions of a naphtha and methanol coupled aromatic hydrocarbon preparation reaction zone of the naphtha and methanol coupled aromatic hydrocarbon preparation reactor are: an apparent linear velocity of gas of 1.0 m/s, a reaction temperature of 580°C, a reaction pressure of 150 kPa, and a bed density of 350 kg/m³.

Regeneration gas is air.

Process conditions of a regeneration zone of a regenerator are: an apparent linear velocity of gas of 1.0 m/s, a regeneration temperature of 680°C, a regeneration pressure of 150 kPa, and a bed density of 350 kg/m³.

A riser reactor raw material comprises water vapor and low-carbon alkanes separated from the product gas stream, wherein a water vapor content in the riser reactor raw material is 40wt%.

Process conditions of a riser reactor are: an apparent linear velocity of gas of 7.0 m/s, a temperature of 650°C, a pressure of 150 kPa, and a bed density of 80 kg/m³.

Abed reactor raw material is C₄ and C₅ hydrocarbons separated from the product gas stream.

Process conditions of a light hydrocarbon aromatization reaction zone are: an apparent linear velocity of gas of 1.0 m/s, a reaction temperature of 610°C, a reaction pressure of 150 kPa, and a bed density of 350 kg/m³.

A carbon content in a spent catalyst is 1.5wt%, and a carbon content in a regenerated catalyst is 0.5wt%.

The naphtha raw material entering the naphtha and methanol coupled aromatic hydrocarbon preparation reactor has a per-pass conversion rate of 72wt%.

The product is composed of 71wt% BTX, 9wt% low-carbon olefin, 4wt% hydrogen, 3wt% low-carbon alkane, 6wt% combustible gas, 6wt% heavy aromatic hydrocarbon and 1.0wt% coke. A content of p-xylene in the mixed xylene in the product is 65wt%.

The above are only several examples of the present application, and are not intended to limit the present application in any form. Although the present application is disclosed in the preferred examples above, the preferred examples are not intended to limit the present application. The changes or modifications made by those skilled in the art by using the technical contents disclosed above without departing from the scope of the technical solution of the present application are equivalent to equivalent embodiments, and all fall within the scope of the technical solution of the present application.

## Claims

1. A device for preparing aromatic hydrocarbons by coupling naphtha and methanol, wherein the device comprises a light hydrocarbon aromatization reactor and a naphtha and methanol coupled aromatic hydrocarbon preparation reactor; wherein, the light hydrocarbon aromatization reactor is used for introducing raw materials and a high-temperature catalyst; and at least one outlet of the light hydrocarbon aromatization reactor is connected to the naphtha and methanol coupled aromatic hydrocarbon preparation reactor for conveying the catalyst and generated light hydrocarbon aromatization product gas to the naphtha and methanol coupled aromatic hydrocarbon preparation reactor; and
the naphtha and methanol coupled aromatic hydrocarbon preparation reactor is used for introducing naphtha and methanol and allowing the naphtha to make contact with the catalyst from the light hydrocarbon aromatization reactor to generate a BTX-containing product gas stream after reaction; and subjecting the methanol to a methylation reaction with benzene and toluene in the product gas stream to generate p-xylene.

2. The device for preparing aromatic hydrocarbons by coupling naphtha and methanol according to claim 1, wherein the naphtha and methanol coupled aromatic hydrocarbon preparation reactor is at least divided into a first gas-solid separation zone and a naphtha and methanol coupled aromatic hydrocarbon preparation reaction zone from top to bottom, and the two zones are communicated; and a naphtha and methanol coupled aromatic hydrocarbon preparation reactor distributor is provided in the naphtha and methanol coupled aromatic hydrocarbon preparation reaction zone, which comprises n sub-distributors, serial numbers of the sub-distributors are 1 to n sequentially from bottom to top, n≥2, wherein, a 1^{st} sub-distributor is used for introducing a naphtha raw material, and a 2^{nd} sub-distributor to an n^{th} sub-distributor are used for introducing a methanol raw material.

3. The device for preparing aromatic hydrocarbons by coupling naphtha and methanol according to claim 2, wherein n≤10.

4. The device for preparing aromatic hydrocarbons by coupling naphtha and methanol according to claim 2, wherein a gas-solid separation device I and a gas collection chamber I are provided in the first gas-solid separation zone; a gas outlet of the gas-solid separation device I is communicated with the gas collection chamber I; and an outlet of the gas collection chamber I is communicated with a product gas conveying pipe I, and the product gas conveying pipe I is used for outputting the BTX-containing product gas stream after gas-solid separation to a downstream working section.

5. The device for preparing aromatic hydrocarbons by coupling naphtha and methanol according to claim 4, wherein the gas collection chamber I is located on an inner top portion of a naphtha and methanol coupled aromatic hydrocarbon preparation reactor shell.

6. The device for preparing aromatic hydrocarbons by coupling naphtha and methanol according to claim 4, wherein the gas-solid separation device I is one or more groups of gas-solid cyclone separators, and each group of gas-solid cyclone separators comprises a first-stage gas-solid cyclone separator and a second-stage gas-solid cyclone separator.

7. The device for preparing aromatic hydrocarbons by coupling naphtha and methanol according to claim 1, wherein the light hydrocarbon aromatization reactor is at least divided into a second gas-solid separation zone and a light hydrocarbon aromatization reaction zone from top to bottom, and the two zones are communicated to form a bed reactor; and the second gas-solid separation zone is provided with a gas-solid separation device II and a gas collection chamber II, a gas outlet of the gas-solid separation device II is communicated with the gas collection chamber II, and a bed reactor distributor is provided on an inner lower portion of the light hydrocarbon aromatization reaction zone for introducing a bed reactor raw material.

8. The device for preparing aromatic hydrocarbons by coupling naphtha and methanol according to claim 7, wherein the gas collection chamber II is provided on an inner top portion of the bed reactor.

9. The device for preparing aromatic hydrocarbons by coupling naphtha and methanol according to claim 7, wherein the bed reactor raw material comprises C₄ and C₅ hydrocarbons.

10. The device for preparing aromatic hydrocarbons by coupling naphtha and methanol according to claim 7, wherein besides the bed reactor, the light hydrocarbon aromatization reactor further comprises a riser reactor, an outlet end of the riser reactor extends into an inner lower portion of the light hydrocarbon aromatization reaction zone, and a catalyst outlet of the gas-solid separation device II is provided above the riser reactor.

11. The device for preparing aromatic hydrocarbons by coupling naphtha and methanol according to claim 7, wherein an inlet end of the riser reactor is also used for introducing the catalyst and a riser reactor raw material.

12. The device for preparing aromatic hydrocarbons by coupling naphtha and methanol according to claim 7, wherein the second gas-solid separation zone is communicated with the first gas-solid separation zone, and the light hydrocarbon aromatization reaction zone is communicated with the naphtha and methanol coupled aromatic hydrocarbon preparation reaction zone.

13. The device for preparing aromatic hydrocarbons by coupling naphtha and methanol according to claim 7, wherein the gas collection chamber II is communicated with the first gas-solid separation zone through a product gas conveying pipe II.

14. The device for preparing aromatic hydrocarbons by coupling naphtha and methanol according to claim 12, wherein a light hydrocarbon aromatization slide valve is provided on a pipeline connecting the naphtha and methanol coupled aromatic hydrocarbon preparation reaction zone and the light hydrocarbon aromatization reaction zone.

15. The device for preparing aromatic hydrocarbons by coupling naphtha and methanol according to claim 7, wherein a position of an outlet of the light hydrocarbon aromatization reaction zone is higher than a position of an inlet of the naphtha and methanol coupled aromatic hydrocarbon preparation reaction zone.

16. The device for preparing aromatic hydrocarbons by coupling naphtha and methanol according to claim 15, wherein a position of a catalyst inlet of the naphtha and methanol coupled aromatic hydrocarbon preparation reaction zone is located between the 1^{st} sub-distributor and the 2^{nd} sub-distributor.

17. The device for preparing aromatic hydrocarbons by coupling naphtha and methanol according to claim 7, wherein the gas-solid separation device II is a gas-solid cyclone separator.

18. The device for preparing aromatic hydrocarbons by coupling naphtha and methanol according to claim 1, wherein the device further comprises a regenerator, and at least one inlet of the light hydrocarbon aromatization reactor is connected to the regenerator for acquiring a high-temperature regenerated catalyst generated by the regenerator.

19. The device for preparing aromatic hydrocarbons by coupling naphtha and methanol according to claim 18, wherein the regenerator is communicated with an inlet end of a riser reactor of the light hydrocarbon aromatization reactor.

20. The device for preparing aromatic hydrocarbons by coupling naphtha and methanol according to claim 18, wherein the regenerator is at least divided into a third gas-solid separation zone and a regeneration zone from top to bottom, and the two zones are communicated; the third gas-solid separation zone is provided with a regenerator gas-solid separation device and a regenerator gas collection chamber; a gas outlet of the regenerator gas-solid separation device is communicated with the regenerator gas collection chamber; a flue gas conveying pipe is provided on the regenerator gas collection chamber; and a regenerator distributor is provided on an inner lower portion of the regeneration zone for introducing regeneration gas.

21. The device for preparing aromatic hydrocarbons by coupling naphtha and methanol according to claim 20, wherein the regeneration zone sequentially passes through a regenerator stripper and a regenerated slide valve to be connected to the riser reactor; and an inlet pipe of the regenerator stripper extends into a regenerator shell and is located above the regenerator distributor, and a catalyst outlet end of the regenerator gas-solid separation device is located above an opening end of the inlet pipe of the regenerator stripper.

22. The device for preparing aromatic hydrocarbons by coupling naphtha and methanol according to claim 20, wherein the regenerator gas collection chamber is located on an inner top portion of the regenerator shell.

23. The device for preparing aromatic hydrocarbons by coupling naphtha and methanol according to claim 20, wherein the regenerator gas-solid separation device is one or more groups of gas-solid cyclone separators, and each group of gas-solid cyclone separators comprises a first-stage gas-solid cyclone separator and a second-stage gas-solid cyclone separator.

24. The device for preparing aromatic hydrocarbons by coupling naphtha and methanol according to claim 1, wherein at least one outlet of the naphtha and methanol coupled aromatic hydrocarbon preparation reactor is also connected with an inlet of a regenerator for introducing a spent catalyst generated by a reaction of the naphtha and methanol coupled aromatic hydrocarbon preparation reactor into the regenerator, and the regenerator is used for introducing regeneration gas to convert the spent catalyst into a regenerated catalyst.

25. The device for preparing aromatic hydrocarbons by coupling naphtha and methanol according to claim 2, wherein the naphtha and methanol coupled aromatic hydrocarbon preparation reaction zone sequentially passes through a reactor stripper, a spent slide valve and a spent agent conveying pipe to be connected to an inlet of a regenerator; an inlet pipe of the reactor stripper extends into a naphtha and methanol coupled aromatic hydrocarbon preparation reactor shell and is located above the 1^{st} distributor, and a catalyst outlet end of the reactor gas-solid separation device is located above an opening end of an inlet pipe of the reactor stripper.

26. The device for preparing aromatic hydrocarbons by coupling naphtha and methanol according to claim 25, wherein an inlet of the regenerator is located in a regeneration zone and is provided on a regenerator shell.

27. A method for preparing aromatic hydrocarbons by coupling naphtha and methanol, wherein the method comprises: preparing aromatic hydrocarbons by using the device for preparing aromatic hydrocarbons by coupling naphtha and methanol according to any one of claims 1 to 6 and a catalyst.

28. The method for preparing aromatic hydrocarbons by coupling naphtha and methanol according to claim 27, wherein the catalyst is a metal molecular sieve bifunctional catalyst.

29. The method for preparing aromatic hydrocarbons by coupling naphtha and methanol according to claim 28, wherein the metal molecular sieve bifunctional catalyst is a metal modified HZSM-5 zeolite molecular sieve; metal for the metal modification is selected from at least one of La, Zn, Ga, Fe, Mo and Cr; and a method for the metal modification comprises: placing the HZSM-5 zeolite molecular sieve in a metal salt solution, soaking, drying and roasting to obtain the metal modified HZSM-5 zeolite molecular sieve.

30. The method for preparing aromatic hydrocarbons by coupling naphtha and methanol according to claim 27, wherein the method comprises the following steps of:
introducing raw materials and a high-temperature catalyst into the light hydrocarbon aromatization reactor to generate the light hydrocarbon aromatization product gas;
introducing naphtha and the catalyst from the light hydrocarbon aromatization reactor into the naphtha and methanol coupled aromatic hydrocarbon preparation reactor to generate a BTX-containing product gas stream; and
introducing methanol into the naphtha and methanol coupled aromatic hydrocarbon preparation reactor to be subjected to a methylation reaction with benzene and toluene in the BTX-containing product gas stream to generate p-xylene.

31. The method for preparing aromatic hydrocarbons by coupling naphtha and methanol according to claim 30, wherein the method further comprises: removing a spent catalyst contained in all gas streams generated in the naphtha and methanol coupled aromatic hydrocarbon preparation reactor through the gas-solid separation device I, and allowing the gas streams to enter the gas collection chamber I and then enter the downstream working section through the product gas conveying pipe I.

32. The method for preparing aromatic hydrocarbons by coupling naphtha and methanol according to claim 30, wherein the light hydrocarbon aromatization product gas comprises components of BTX, low-carbon olefins and H₂.

33. The method for preparing aromatic hydrocarbons by coupling naphtha and methanol according to claim 30, wherein besides the BTX, the BTX-containing product gas stream further comprises low-carbon olefins, hydrogen, low-carbon alkanes, combustible gas, heavy aromatic hydrocarbons and unconverted naphtha.

34. The method for preparing aromatic hydrocarbons by coupling naphtha and methanol according to claim 33, wherein the low-carbon olefins refer to ethylene and propylene; the low-carbon alkanes refer to ethane and propane; the combustible gas comprises methane and CO; and the heavy aromatic hydrocarbons refer to aromatic hydrocarbons with a number of carbon atoms in a molecule greater than or equal to 9.

35. The method for preparing aromatic hydrocarbons by coupling naphtha and methanol according to claim 30, wherein the naphtha is selected from at least one of direct coal liquefaction naphtha, indirect coal liquefaction naphtha, straight-run naphtha and hydrocracking naphtha.

36. The method for preparing aromatic hydrocarbons by coupling naphtha and methanol according to claim 35, wherein the naphtha also contains unconverted naphtha separated from the product gas stream, and the unconverted naphtha comprises main components of C₄-C₁₂ linear-chain and branched-chain aliphatic hydrocarbons and naphthenic hydrocarbons.

37. The method for preparing aromatic hydrocarbons by coupling naphtha and methanol according to claim 31, wherein a carbon content in the spent catalyst is 1.0wt% to 3.0wt%.

38. The method for preparing aromatic hydrocarbons by coupling naphtha and methanol according to claim 30, wherein process conditions of the naphtha and methanol coupled aromatic hydrocarbon preparation reaction zone in the naphtha and methanol coupled aromatic hydrocarbon preparation reactor are: an apparent linear velocity of gas of 0.5 m/s to 2.0 m/s, a reaction temperature of 500°C to 600°C, a reaction pressure of 100 kPa to 500 kPa, and a bed density of 150 kg/m³ to 700 kg/m³.

39. The method for preparing aromatic hydrocarbons by coupling naphtha and methanol according to claim 30, wherein the light hydrocarbon aromatization product gas enters a gas-solid separation device II to remove a catalyst contained in the light hydrocarbon aromatization product gas, then enters a gas collection chamber II, and enters the first gas-solid separation zone of the naphtha and methanol coupled aromatic hydrocarbon preparation reactor through a product gas conveying pipe II; and
the catalyst in the light hydrocarbon aromatization reaction zone enters the naphtha and methanol coupled aromatic hydrocarbon preparation reactor through a light hydrocarbon aromatization slide valve.

40. The method for preparing aromatic hydrocarbons by coupling naphtha and methanol according to claim 30, wherein process conditions of the light hydrocarbon aromatization reaction zone in the light hydrocarbon aromatization reactor are: an apparent linear velocity of gas of 0.5 m/s to 2.0 m/s, a reaction temperature of 550°C to 665°C, a reaction pressure of 100 kPa to 500 kPa, and a bed density of 150 kg/m³ to 700 kg/m³.

41. The method for preparing aromatic hydrocarbons by coupling naphtha and methanol according to claim 30, wherein the method further comprises: introducing regeneration gas and a spent catalyst into a regenerator to obtain a high-temperature regenerated catalyst, and conveying the high-temperature regenerated catalyst to the light hydrocarbon aromatization reactor.

42. The method for preparing aromatic hydrocarbons by coupling naphtha and methanol according to claim 41, wherein the regeneration gas is introduced into a regeneration zone of the regenerator through a regenerator distributor.

43. The method for preparing aromatic hydrocarbons by coupling naphtha and methanol according to claim 41, wherein the regeneration gas is selected from at least one of oxygen, air and oxygen-enriched air.

44. The method for preparing aromatic hydrocarbons by coupling naphtha and methanol according to claim 41, wherein a carbon content in the spent catalyst is 1.0wt% to 3.0wt%.

45. The method for preparing aromatic hydrocarbons by coupling naphtha and methanol according to claim 41, wherein a carbon content in the regenerated catalyst is less than or equal to 0.5wt%.

46. The method for preparing aromatic hydrocarbons by coupling naphtha and methanol according to claim 41, wherein process conditions of the regeneration zone of the regenerator are: an apparent linear velocity of gas of 0.5 m/s to 2.0 m/s, a regeneration temperature of 600°C to 750°C, a regeneration pressure of 100 kPa to 500 kPa, and a bed density of 150 kg/m³ to 700 kg/m³.

47. The method for preparing aromatic hydrocarbons by coupling naphtha and methanol according to claim 41, wherein coke on the spent catalyst reacts with the regeneration gas to generate flue gas, and the flue gas enters a third gas-solid separation zone to remove a regenerated catalyst contained in the flue gas.

48. The method for preparing aromatic hydrocarbons by coupling naphtha and methanol according to claim 47, wherein the flue gas enters the third gas-solid separation zone to remove the regenerated catalyst contained in the flue gas, which specifically comprises that: the flue gas enters a regenerator gas-solid separation device first, and after the regenerated catalyst contained in the flue gas is removed, the flue gas passes through a regenerator gas collection chamber and a flue gas conveying pipe to enter a downstream working section.

49. The method for preparing aromatic hydrocarbons by coupling naphtha and methanol according to claim 41, wherein the regenerated catalyst enters the light hydrocarbon aromatization reactor through a regenerator stripper and a regenerated slide valve.

50. The method for preparing aromatic hydrocarbons by coupling naphtha and methanol according to claim 30, wherein the method further comprises: introducing a riser reactor raw material into an inlet end of a riser reactor of the light hydrocarbon aromatization reactor; and introducing the regenerated catalyst into the riser reactor through a regenerator stripper and a regenerated slide valve, converting the riser reactor raw material into the BTX-containing stream under an action of the regenerated catalyst, and allowing the BTX-containing stream to enter an inner lower portion of a light hydrocarbon aromatization reaction zone in a bed reactor through an outlet end of the riser reactor.

51. The method for preparing aromatic hydrocarbons by coupling naphtha and methanol according to claim 50, wherein the riser reactor raw material comprises water vapor and the low-carbon alkanes separated from the product gas stream.

52. The method for preparing aromatic hydrocarbons by coupling naphtha and methanol according to claim 50, wherein a water vapor content in the riser reactor raw material is 0wt% to 80wt%.

53. The method for preparing aromatic hydrocarbons by coupling naphtha and methanol according to claim 50, wherein process conditions of the riser reactor are: an apparent linear velocity of gas of 3.0 m/s to 10.0 m/s, a temperature of 580°C to 700°C, a pressure of 100 kPa to 500 kPa, and a bed density of 50 kg/m³ to 150 kg/m³.

54. The method for preparing aromatic hydrocarbons by coupling naphtha and methanol according to claim 50, wherein the method further comprises: introducing a catalyst into an inlet end of the riser reactor of the light hydrocarbon aromatization reactor, and allowing the catalyst to enter the bed reactor through the riser reactor.

55. The method for preparing aromatic hydrocarbons by coupling naphtha and methanol according to claim 54, wherein the method further comprises: introducing a bed reactor raw material into the light hydrocarbon aromatization reaction zone through a bed reactor distributor to make contact with the catalyst from the riser reactor to generate the light hydrocarbon aromatization product gas.

56. The method for preparing aromatic hydrocarbons by coupling naphtha and methanol according to claim 54, wherein the bed reactor raw material comprises C₄ and C₅ hydrocarbons.

57. The method for preparing aromatic hydrocarbons by coupling naphtha and methanol according to claim 56, wherein the C₄ and C₅ hydrocarbons come from C₄ and C₅ hydrocarbons separated from the product gas stream.

58. The method for preparing aromatic hydrocarbons by coupling naphtha and methanol according to claim 50, wherein the BTX-containing stream comprises components of BTX, low-carbon olefins and H₂.

59. The method for preparing aromatic hydrocarbons by coupling naphtha and methanol according to claim 30, wherein the method further comprises:
introducing the spent catalyst in the naphtha and methanol coupled aromatic hydrocarbon preparation reaction zone into the reactor stripper, and allowing the spent catalyst to enter a downstream area through a spent slide valve and a spent agent conveying pipe after stripping.

60. The method for preparing aromatic hydrocarbons by coupling naphtha and methanol according to claim 59, wherein the downstream area is a regenerator.
